(19) Europäisches Patentamt
European Patent Office
Office européen des brevets

(11) Numéro de publication: **0 012 072**
**B1**

(12) # FASCICULE DE BREVET EUROPEEN

(45) Date de publication du fascicule du brevet: **16.05.84**

(21) Numéro de dépôt: **79400905.0**

(22) Date de dépôt: **23.11.79**

(51) Int. Cl.$^3$: **C 07 D 305/14,** A 61 K 31/365
// C07D309/12, C07D407/12

(54) **Bêta-lactones dérivées de l'acide 2-hydroxy cyclopentane carboxylique, procédé pour leur préparation et compositions pharmaceutiques les renfermant.**

(30) Priorité: **05.12.78 FR 7834183**

(43) Date de publication de la demande:
**11.06.80 Bulletin 80/12**

(45) Mention de la délivrance du brevet:
**16.05.84 Bulletin 84/20**

(84) Etats contractants désignés:
**FR**

(56) Documents cités:
**FR-A-2 344 285**
**FR-A-2 383 936**

(73) Titulaire: **ROUSSEL-UCLAF**
**102, route de Noisy Boîte postale no.9**
**F-93230 Romainville (FR)**

(72) Inventeur: **Buendia, Jean**
**3bis Impasse Emilie**
**F-94170-Le Perreux sur Marne (FR)**
Inventeur: **Vivat, Michel**
**14, Chemin de l'Autostrade**
**F-77400-Lagny (FR)**
Inventeur: **Taliani, Laurent**
**107, Allée Danielle Casanova**
**F-93320-Les Pavillons sous Bois (FR)**

(74) Mandataire: **Petranker, Léon et al**
**boîte postale no 9 102, route de Noisy**
**F-93230 Romainville (FR)**

**EP 0 012 072 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne de nouvelles $\beta$-lactones dérivées de l'acide 2-hydroxy cyclopentane carboxylique, leur procédé de préparation, leur application comme médicaments et les compositions pharmaceutiques les renfermant.

Dans la demande de brevet français 2.344.285 et la demande de certificat d'addition français 2.383.936, la société demanderesse a décrit et revendiqué de nouvelles lactones dérivées du cyclopentanol et notamment la lactone de l'acide (1RS, 2SR, 5RS, 3'SR) (1'E) 2-hydroxy-5-(3'$\alpha$-hydroxy 1'-octényl) cyclopentane carboxylique.

Les produits de la présente invention présentent, par rapport aux produits de l'art antérieur, une activité améliorée et l'avantage de se présenter sous une forme cristallisée qui rend beaucoup plus facile la purification et la mise sous forme pharmaceutique des produits. Les produits de l'art antérieur qui possèdent une activité intéressante se présente en effet sous forme d'huiles.

L'invention a pour objet les produits de formule générale I:

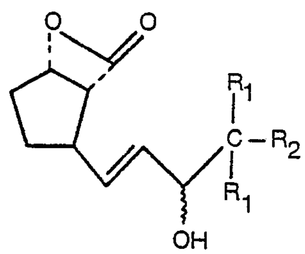

I

dans laquelle $R_1$ représente un atome d'hydrogène ou un radical méthyle et $R_2$ représente: un radical —X—Ar dans lequel X représente un atome d'oxygène ou un radical méthylène et Ar représente un radical phényle non substitué ou substitué par un ou plusieurs radicaux choisis parmi les atomes d'halogène et le radical trifluorométhyle, le trait ondulé signifie que la liaison peut se trouver dans l'une ou l'autre des configurations possibles.

Le radical Ar peut être substitué par un ou plusieurs atomes de fluor, de chlore, de brome ou d'iode et/ou par un radical trifluorométhyle.

Parmi les produits de formule I on peut citer notamment:

la lactone de l'acide (1RS, 2SR, 5RS, 3'SR) (1'E) 2-hydroxy 5-/3'-hydroxy 4'-phényloxy 1'-butényl/cyclopentane-1-carboxylique et,

la lactone de l'acide (1RS, 2SR, 5RS, 3'RS) (1'E) 2-hydroxy 5-/3'-hydroxy 4'-phényloxy 1'-butényl/cyclopentane-1-carboxylique.

L'invention a également pour objet un procédé de préparation des produits de formule générale I telle que définie ci-dessus, caractérisé en ce que l'on soumet un produit de formule II:

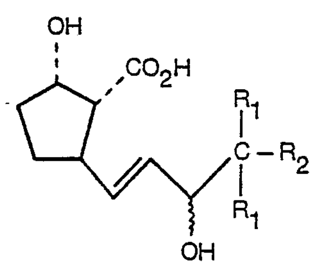

II

dans laquelle $R_1$ et $R_2$ ont la signification donnée ci-dessus à l'action d'un réactif de formation de dérivés fonctionnels des acides, choisi dans le groupe formé par le chlorure de tosyle, les chloroformates d'alcoyle, les dicycloalcoylcarbodiimides, les dialcoylcarbodiimides et le chlorure de thionyle.

Le dérivé fonctionnel obtenu réagit ensuite avec le radical hydroxyle pour former le cycle lactonique du produit cherché.

Dans un mode préférentiel d'exécution du procédé ci-dessus, le réactif de formation de dérivés fonctionnels des acides auquel on soumet le produit de formule II est le chlorure de tosyle, pour former un dérivé fonctionnel qui est l'anhydride mixte.

On opère alors de préférence en présence d'une base telle qu'un carbonate de métal alcalin ou une base organique telle que le méthylmorpholine, la pyridine, une trialcoylamine telle que la triéthylamine, ou le diazabicyclooctane.

On opère également de préférence en présence d'une base choisie dans le même groupe lorsque

l'on prépare un dérivé fonctionnel en utilisant un chloroformiate d'alcoyle tel que le chloroformiate d'isobutyle ou lorsque l'on utilise le chlorure de thionyle.

L'invention a également pour objet un procédé de préparation des produits de formule I tels que définis ci-dessus, caractérisé en ce que l'on soumet le produit de formule III

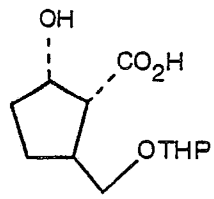

III

dans laquelle THP représente un radical tétrahydropyrannyle, à l'action d'un réactif de formation de dérivés fonctionnels des acides pour obtenir un produit de formule IV

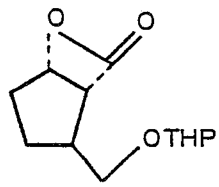

IV

produit que l'on traite par un acide pour obtenir un produit de formule V

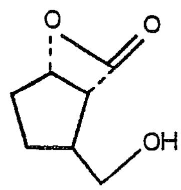

V

produit que l'on traite par un agent d'oxydation pour obtenir un produit de formule VI

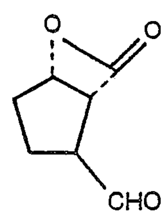

VI

produit que l'on traite par un phosphonate de formule:

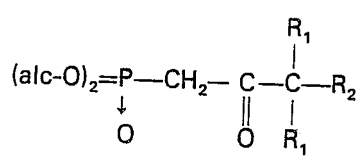

VII

dans laquelle $R_1$ et $R_2$ ont la signification donnée ci-dessus et alc représente un radical alkyle ayant de 1 à 4 atomes de carbone, en présence d'une base forte, pour obtenir un produit de formule VII:

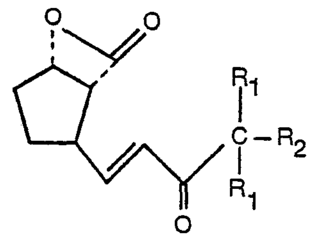

produit que l'on traite par un agent réducteur pour obtenir un produit de formule I.

La transformation du produit de formule III en produit de formule IV est effectuée dans les mêmes conditions que la transformation des produits de formule II en produit de formule I. On utilise de préférence l'anhydride mixte préparé avec le chlorure de tosyle en opérant en présence de diazabicyclooctane.

L'acide que l'on utilise de préférence pour transformer les produits de formule IV en produits de formule V est de préférence l'acide oxalique. On peut cependant utiliser les acides chlorhydrique, acétique ou sulfurique.

L'agent d'oxydation que l'on utilise de préférence pour passer des produits de formule V aux produits de formule VI est le complexe oxyde de chrome dans la pyridine. On peut cependant utiliser l'oxyde de chrome dans la triéthylamine ou dans une collidine.

Les phosphonates que l'on utilise pour préparer les produits de formule VII à partir des produits de formule VI sont de préférence ceux dans lesquels le substituant alc représente un radical méthyle. On peut cependant utiliser ceux dans lesquels alc représente un radical éthyle, propyle ou butyle.

La base forte que l'on utilise de préférence est l'hydrure de sodium mais on peut également utiliser l'amidure de sodium, le tert-amylate de sodium ou le butyl lithium.

L'agent réducteur que l'on utilise pour transformer les produits de formule VII en produits de formule I est de préférence le borohydrure de zinc. On peut cependant utiliser le borohydrure de sodium, le tris méthoxy borohydrure de sodium, ou le tris (sec-butyl, borohydrure de lithium.

Les produits de formule I et II qui comportent un groupement hydroxyle pouvant se trouver dans l'une ou l'autre des deux positions possibles, $\alpha$ et $\beta$ sur l'atome de carbone auquel ce groupe est lié, se présentent donc sous forme d'un mélange qui peut être séparé par les méthodes physiques usuelles, en particulier la chromatographie.

Les produits de formule I peuvent exister sous formes racémiques ou optiquement actives; les isomères actifs peuvent être séparés par les méthodes habituellement employées.

Les produits objet de la présente demande manifestent en pharmacologie une activité hypotensive.

On trouvera plus loin dans la partie expérimentale, les résultats pharmacologiques obtenus avec les produits de l'invention.

Les propriétés pharmacologiques des produits de l'invention les rendent aptes à être utilisés comme médicaments, notamment dans le traitement de l'hypertension et des troubles circulatoires.

La présente invention a donc également pour objet, à titre de médicaments, les composés thérapeutiquement actifs de formule I, tels que définis ci-dessus et notamment la lactone de l'acide (1RS, 2SR, 5RS, 3'SR) (1'E) 2-hydroxy 5-/3'-hydroxy 4'-phényloxy 1'-butényl/cyclopentane-1-carboxylique et la lactone de l'acide (1RS, 2SR, 5RS, 3'RS) (1'E)-2-hydroxy 5-/3'-hydroxy 4'-phényloxy 1'-butényl/cyclopentane-1-carboxylique.

L'invention s'étend aux compositions pharmaceutiques renfermant, comme principe actif, au moins un des médicaments définis ci-dessus.

Ces compositions pharmaceutiques peuvent être administrées par voie buccale, rectale, par voie parentérale, par voie locale.

Elles peuvent être solides ou liquides et se présenter sous les formes pharmaceutiques couramment utilisées en médecine humaine, comme, par exemple, les comprimés, simples ou dragéifiés, les gélules, les granulés, les suppositoires, les préparations injectables; elles sont préparés selon les méthodes usuelles. Le ou les principes actifs peuvent y être incorporés à des excipients habituellement employés dans ces compositions pharmaceutiques, tels que la talc, la gomme arabique, le lactose, l'amidon, le stéarate de magnésium, le beurre de cacao, les véhicules aqueux ou non.

La dose administrée est variable selon l'affection traitée, le sujet en cause, la voie d'administration et les produits considérés. Elle peut être, par exemple, comprise entre 0,5 mg et 200 mg avec les produits des exemples 1 ou 2 décrits dans la partie expérimentale, administrés par exemple par voie injectable, chez l'homme.

Les produits de formule II utilisés au départ du procédé peuvent être préparés comme suit:

On traite un produit de formule IX:

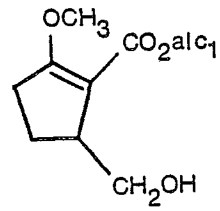

IX

dans laquelle $alc_1$ représente un radical alkyle ayant de 1 à 4 atomes de carbone par un agent d'oxydation, tel que l'oxyde de chrome dans la pyridine, pour obtenir un produit de formule X

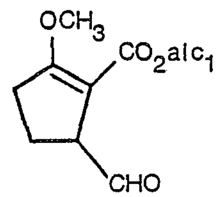

X

produit que l'on traite par un phosphonate de formule

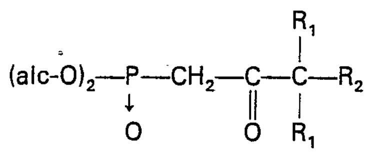

dans laquelle $R_1$ et $R_2$ ont la signification précédente et alc représente un radical alkyle ayant de 1 à 4 atomes de carbone, en présence d'une base forte telle que l'hydrure de sodium, pour obtenir un produit de formule XI

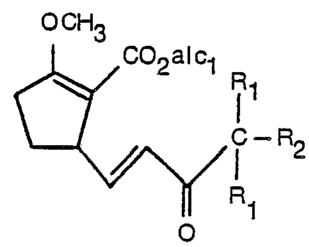

XI

produit que l'on traite par un agent réducteur, tel que le borohydrure de zinc, pour obtenir un produit de formule XII

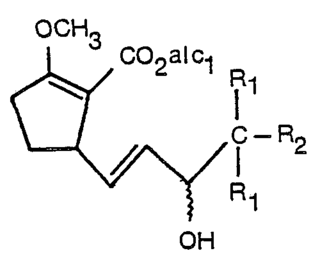

XII

produit que l'on sépare éventuellement en ses isomères, et mélange ou produits séparés que l'on traite par un acide, tel que l'acide oxalique, pour obtenir un produit de formule XIII

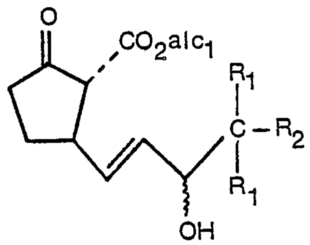

XIII

produit que l'on traite par un agent réducteur, tel que le tris (sec-butyl) borohydrure de sodium, pour obtenir un produit de formule XIV:

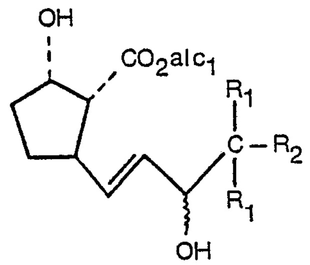

XIV

produit que l'on traite par une base, telle que la soude, puis par un acide, tel que l'acide chlorhydrique, dilué pour obtenir un produit de formule II.

Les produits de formule III sont préparés en faisant agir sur un produit de formule XV:

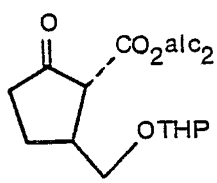

XV

dans laquelle $alc_2$ représente un radical alkyle ayant de 1 à 4 atomes de carbone et THP représente un radical $\alpha$-tétrahydropyrannyle, un agent réducteur, tel que le tris (sec-butyl) borohydrure de lithium, pour obtenir un produit de formule XVI:

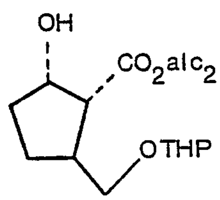

XVI

que l'on traite par une base, telle que la soude, puis par un acide, tel que l'acide chlorhydrique dilué, pour obtenir un produit de formule III.

Les produits de formules IX et XV sont décrits dans le brevet français 2 332 971.

Des exemples de telles préparations sont données plus loin dans la partie expérimentale.

Les exemples suivants illustrent l'invention sans toutefois la limiter.

Exemple 1

Lactone de l'acide (1RS, 2SR, 5RS, 3'RS) (1'E) 2-hydroxy 5-/3'hydroxy 4'-phényloxy 1'-buténylcyclopentane-1-carboxylique.

On mélange, sous agitation, 71 mg d'acide (1RS, 2SR, 5RS, 3'RS) (1'E)2-hydroxy 5-/3'-hydroxy 4'-phényloxy 1'-buténylcyclopentane-carboxylique, 2,6 ml de chloroforme et 55 mg de chlorure de tosyle. Après dissolution, on ajoute 108 mg de diazabicyclooctane et laisse sous agitation pendant 1 heure à température ambiante. On filtre, lave le filtrat à l'acide chlorhydrique 1N, extrait la solution acide à l'acétate d'éthyle, sèche les phases organiques réunies et évapore le solvant sous pression réduite. On purifie le résidu par chromatographie sur silice en éluant au mélange chlorure de méthylène-acétate d'éthyle (95—5) et obtient 42 mg de produit attendu. Rf = 0,25. F = 83°C.

Spectre IR ($CHCl_3$)

| | |
|---|---|
| OH | 3580 $cm^1$ |
| lactone | 1821 $cm^{-1}$ |
| C=C | 968 $cm^{-1}$ |
| bandes aromatiques | 1598—1586—1495 $cm^{-1}$ |

Spectre RMN ($CDCl_3$, 90 MHz)

(c)

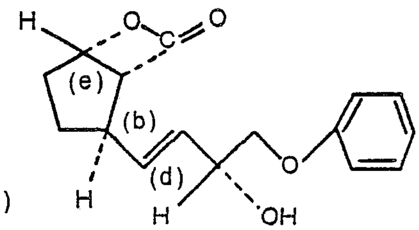

(a)

(f)

(a): 4,53 à 4,96 ppm
(b): 3,53 à 3,99 ppm
(c): 3,31—3,35 et 3,39 ppm
(d): 2,99 ppm (multiplet)
(e): 2,43 à 2,74 ppm
(f): 2,09 ppm.

## Exemple 2

Lactone de l'acide (1RS, 2SR, 5RS, 3'SR) (1'E)-2-hydroxy 5-/3'-hydroxy 4'-phényloxy 1'-buténényl/cyclopentane-1-carboxylique.

On opère de manière analogue à celle décrite à l'exemple 1, au départ de l'acide (1RS, 2SR, 5RS, 3'SR) (1'E) 2-hydroxy 5-/3'-hydroxy 4'-phényloxy 1'-buténényl/cyclopentane-1-carboxylique et obtient après purification par chromatographie sur silice dans le mélange chlorure de méthylène-acétate d'éthyle (95—5), le produit attendu. F = 90°C.

Les acides (1RS, 2SR, 5RS, 3'RS) (1'E) 2-hydroxy 5-/3'-hydroxy 4'-phényloxy 1'-buténényl/cyclopentane carboxylique et (1RS, 2SR, 5RS, 3'SR) (1'E) 2-hydroxy 5-/3'-hydroxy 4'-phényloxy 1'-buténényl/cyclopentane carboxylique utilisés respectivement au départ des exemples 1 et 2 ont été préparés comme suit:

### Stade A

(5RS) (1'E) 2-méthoxy 5-/3'-oxo 4'-phényloxy 1'-buténényl/1-cyclopentène carboxylate de méthyle.

On ajoute lentement, sous agitation et sous atmosphère inerte, 65 mg d'hydrure de sodium à 60% dans l'huile minérale, dans 5 cm3 de diméthoxyéthane, ajoute 413 mg de 2-oxo 3-(3'-phényloxy)propyl phosphonate de diméthyle dans 5 cm3 de diméthoxyéthane, agite pendant deux heures à 20°C puis ajoute à la suspension obtenue 290 mg de (RS) 2-méthoxy 5-formyl 1-cyclopentène carboxylate de méthyle en solution dans 5 cm3 de diméthoxyéthane.

On agite à 20°C pendant 24 heures, verse sur une solution aqueuse glacée de phosphate monosodique, extrait à l'acétate d'éthyle, lave la phase organique à l'eau, sèche et évapore à sec. On chromatographie le résidu sur silice dans le mélange cyclohexane-acétate d'éthyle (1—1) à 0,2% de triéthylamine et obtient 174 mg de produit attendu que l'on cristallise dans le mélange éther-éther isopropylique. F = 90°C.

### Stade B

(3RS, 3'RS) (1'E) 2-méthoxy 5-/3'-hydroxy 4'-phényloxy 1'-buténényl/1-cyclopentène carboxylate de méthyle et (5RS, 3'SR) (1'E) 2-méthoxy 5-/3'-hydroxy 4'-phényloxy 1'-buténényl/1-cyclopentène carboxylate de méthyle.

On dissout sous agitation, 392 mg de produit obtenu au stade A dans 8 ml d'un mélange méthanol-eau (4—1), et introduit 66 mg d'hydroborure de potassium. On maintient la suspension sous agitation pendant 2 heures à température ambiante, ajoute 4 ml d'une solution aqueuse saturée de phosphate monosodique et évapore le solvant sous pression réduite. On extrait à l'acétate d'éthyle, sèche les phases organiques et les amène à sec sous pression réduite. On purifie le résidu par chromatographique sur silice en éluant au mélange chlorure de méthylèneacétate d'éthyle (90—10) à

0,1% de triéthylamine, 110 mg d'isomère (3'RS) (Rf = 0,35) et 130 mg d'isomère (3'SR) (RF = 0,40) sont obtenus.

Stade C

(1RS, 5RS, 3'RS) (1'E) 2-oxo 5-/3'-hydroxy 4'-phényloxy 1'-butényl/cyclopentane carboxylate de méthyle.

On dissout, sous agitation, 110 mg d'isomère (3'RS) obtenu au stade B dans 12 ml d'un mélange méthanol-eau (1—2) puis on introduit 175 mg d'acide oxalique et laisse le mélange réactionnel pendant 4 heures à température ambiante et sous agitation. On élimine le solvant sous pression réduite, extrait au chloroforme, sèche la phase organique et l'amène à sec sous pression réduite. On purifie le résidu par chromatographie sur silice en éluant dans le mélange chlorure de méthylène-acétate d'éthyle (85—15), 84 mg de produit sont obtenus. (Rf = 0,35).

Stade D

(1RS, 2SR, 5RS, 3'RS) (1'E) 2-hydroxy 5-/3'-hydroxy 4'-phényloxy 1'-butényl/cyclopropane carboxylate de méthyle.

On refroidit à —60°C, 1,14 ml d'une solution molaire de L-sélectride dans le tétrahydrofuranne et on introduit en 10 minutes à —60°C, une solution de 116 mg de produit obtenu au stade C dans 3 ml de tétrahydrofuranne. On laisse le mélange réactionnel pendant 1 heure à —60°C sous agitation, le verse sur une solution aqueuse saturée de phosphate monosodique, extrait à l'acétate d'éthyle, sèche sur sulfate de magnésium et amène à sec sous pression réduite. On purifie le résidu par chromatographie sur silice dans le mélange chlorure de méthylène-acétate d'éthyle (85—15), 85 mg de produit sont obtenus. (Rf = 0,13). Le produit est cristallisé dans le mélange éther-éther isopropylique. F = 109°C.

Stade E

Acide (1RS, 2SR, 5RS, 3'RS) (1'E) 2-hydroxy 5-/3'-hydroxy 4'-phényloxy 1'-butényl/cyclopropane carboxylique.

Dans un mélange de 85 mg de produit obtenu au stade D, 1,5 ml de méthanol, on introduit en une fois 1 ml d'hydroxyde de sodium 1N. On laisse le mélange réactionnel pendant 4 heurs sous agitation à température ambiante. On verse dans l'eau et lave à l'acétate d'éthyle. On acidifie la phase aqueuse avec du phosphate acide de sodium, extrait à l'acétate d'éthyle, sèche et concentre à sec. On obtient 71 mg d'acide attendu. F = 146°C.

Stade F

(1RS, 5RS, 3'SR) (1'E) 2-oxo 5-/3'-hydroxy 4'-phényloxy 1'-butényl/cyclopentane carboxylate de méthyle.

On met sous agitation pendant 5 heures à température ambiante, un mélange de 130 mg d'isomère (3'SR) obtenu au stade B, 8 ml d'eau, 6 ml de méthanol et 200 mg d'acide oxalique. A la fin de la réaction, on évapore à sec sous pression réduite, extrait au chloroforme, sèche et concentre à sec sous pression réduite. On purifie le résidu par chromatographie sur silice en éluant au mélange chlorure de méthylène-acétate d'éthyle (8—2) et recueille 100 mg de produit attendu. Rf = 0,35.

Stade G

(1RS, 2SR, 5RS, 3'SR) (1'E) 2-hydroxy 5-/3'-hydroxy 4'-phényloxy 1'-butényl/cyclopentane carboxylate de méthyle.

On refroidit à —60°C, 5,4 ml d'une solution molaire de L-sélectride dans le tétrahydrofuranne et introduit en 10 minutes à cette température, une solution de 540 mg de produit obtenu au stade F dans 12 ml de tétrahydrofuranne. On laisse le mélange réactionnel sous agitation pendant 5 heures à —60°C. On verse sur une solution aqueuse saturée de phosphate monosodique, extrait à l'acétate d'éthyle, sèche et amène à sec sous pression réduite. On purifie le résidu par chromatographie sur silice en éluant au mélange cyclohexane — acétate d'éthyle (4—6) et obtient 340 mg de produit attendu. Rf = 0,2.

Stade H

Acide (1RS, 2SR, 5RS, 3'SR) (1'E) 2-hydroxy 5-/3'-hydroxy 4'-phényloxy 1'-butényl/cyclopentane carboxylique.

On met sous agitation un mélange de 0,34 g de produit obtenu au stade G, 6,8 ml de méthanol, et introduit en une seule fois 4,4 ml d'hydroxyde de sodium 1N. On laisse le mélange réactionnel pendant 4 heures sous agitation à température ambiante et verse dans l'eau. On lave trois fois à l'acetate d'éthyle. On acidifie la phase aqueuse avec du phosphate acide de sodium. On extrait à l'acétate d'éthyle, sèche et amène à sec. On obtient 330 mg de produit attendu.

Etude pharmacologique des produits objet de l'invention.

Activité hypotensive chez le lapin.

Le produit de l'exemple 2 est utilisé en solution dans le sérum physiologique à 10% d'éthanol. On administre les solutions par voie intraveineuse à des lapins anesthésiés à l'uréthane et on mesure la pression carotidienne.

On administre le produit aux doses de 5, 10 et 20 $\mu$g/kg et on mesure l'abaissement de la pression carotidienne.

Ob obtient des résultats suivants:

| Dose | Abaissement |
|---|---|
| 5 $\mu$g/kg | 19% |
| 10 $\mu$g/kg | 33% |
| 20 $\mu$g/kg | 47% |

**Revendications**

1. Les produits de formule générale I

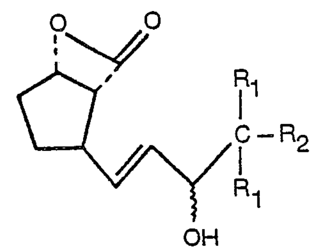

I

dans laquelle $R_1$ représente un atome d'hydrogène ou un radical méthyle et $R_2$ représente un radical —X—Ar dans lequel X représente un atome d'oxygène ou un radical méthylène et Ar représente un radical phényle, non substitué ou substitué par un ou plusieurs radicaux choisis parmi les atomes d'halogène et le radical trifluorométhyle, le trait ondulé signifie que la liaison peut se trouver dans l'une ou l'autre des configurations possibles.

2. La lactone de l'acide (1RS, 2SR, 5RS, 3'RS) (1'E) 2-hydroxy 5-/3'-hydroxy 4'-phényloxy 1'-buténył/cyclopentane-1-carboxylique.

3. La lactone de l'acide (1RS, 2SR, 5RS, 3'SR) (1'E) 2-hydroxy 5-/3'-hydroxy 4'-phényloxy 1'-buténył/cyclopentane-1-carboxylique.

4. Procédé de préparation des produits de formule I telle que définie à la revendication 1, caractérisé en ce que l'on soumet un produit de formule II:

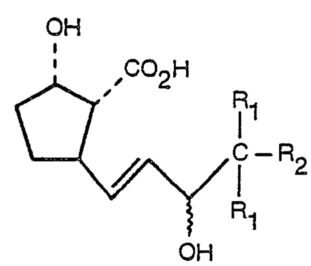

II

dans laquelle $R_1$ et $R_2$ ont la signification donnée à la revendication 1 à l'action d'un réactif de formation de dérivés fonctionnels des acides, choisi dans le groupe formé par le chlorure de tosyle, les chloroformiates d'alcoyle, les dicyclo alcoylcarbodiimides, les dialcoylcarbodiimides et le chlorure de thionyle.

5. Procédé de préparation des produits de formule I telle que définie à la revendication 1, caractérisé en ce que l'on soumet le produit de formule III

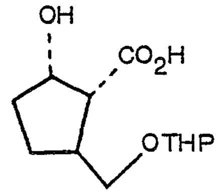

III

dans laquelle THP représente un radical tétrahydropyrannyle, à l'action d'un réactif de formation de dérivés fonctionnels des acides pour obtenir un produit de formule IV

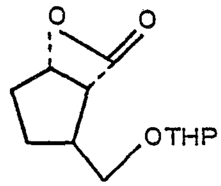

IV

produit que l'on traite par un acide pour obtenir un produit de formule V

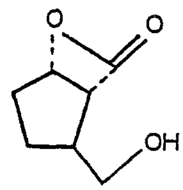

V

produit que l'on traite par un agent d'oxydation pour obtenir un produit de formule VI

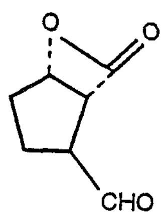

VI

produit que l'on traite par un phosphonate de formule

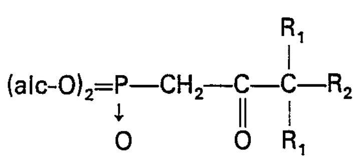

dans laquelle $R_1$ et $R_2$ ont la signification donnée à la revendication 1 et alc représente un radical alkyle ayant de 1 à 4 atomes de carbone, en présence d'une base forte, pour obtenir un produit de formule VII

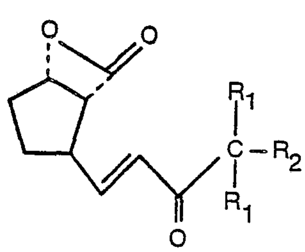

VII

produit que l'on traite par un agent réducteur pour obtenir un produit de formule I.

6. A titre de médicaments les produits répondant à la formule I telle que définie à la revendication 1.

7. A titre de médicaments les produits répondant à la formule I telle que définie à l'une des revendications 2 ou 3.

8. Les compositions pharmaceutiques contenant à titre de principe actif au moins un des médicaments selon l'une des revendications 6 ou 7.

**Patentansprüche**

1. Produkte der allgemeinen Formel I

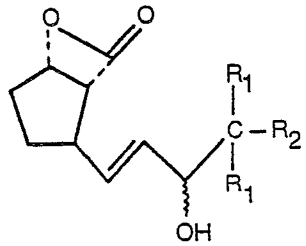

I

worin $R_1$ ein Wasserstoffatom oder einen Methylrest bedeutet und $R_2$ einen —X—Ar-Rest darstellt, worin X ein Sauerstoffatom oder eine Methylengruppe bedeutet und Ar einen unsubstituierten oder durch ein oder mehrere Reste, ausgewählt unter den Halogenatomen und dem Trifluormethylrest, substituierten Phenylrest, darstellt und die gewellte Linie anzeigt, daß die Bindung sich in der einen oder der anderen der möglichen Konfigurationen befinden kann.

2. Lacton der (1RS, 2SR, 5RS, 3'RS)-(1'E)-2-Hydroxy-5-[3'-hydroxy-4'-phenyloxy-1'-butenyl]-cyclopentan-1-carbonsäure.

3. Lacton der (1RS, 2SR, 5RS, 3'SR)-(1'E)-2-Hydroxy-5-[3'-hydroxy-4'-phenyloxy-1'-butenyl]-cyclopentan-1-carbonsäure.

4. Verfahren zur Herstellung der Produkte der Formel I gemäß Anspruch 1, dadurch gekennzeichnet, daß man ein Produkt der Formel II

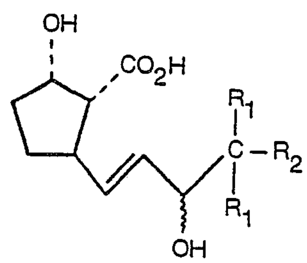

II

worin $R_1$ und $R_2$ die in Anspruch 1 angegebene Bedeutung besitzen, der Einwirkung eines Reagens zur Bildung funktioneller Säurederivate, ausgewählt unter Tosylchlorid, den Alkylchlorformiaten, den Dicycloalkylcarbodiimiden, den Dialkylcarbodiimiden und dem Thionylchlorid, unterzieht.

5. Verfahren zur Herstellung der Produkte der Formel I gemäß Anspruch 1, dadurch gekennzeichnet, daß man das Produkt der Formel III

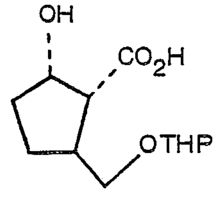

III

worin THP einen Tetrahydropyranylrest bedeutet, der Einwirkung eines Reagens zur Bildung funktioneller Säurederivate unterzieht, um ein Produkt der Formel IV

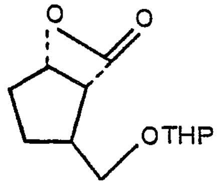

IV

zu erhalten, welches Produkt man mit einer Säure behandelt, um ein Produkt der Formel V

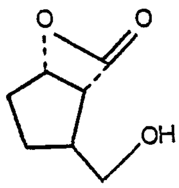

V

zu erhalten, welches Produkt man mit einem Oxidationsmittel behandelt, um ein Produkt der Formel VI

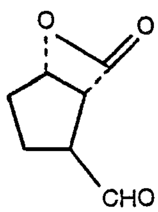

VI

zu erhalten, welches Produkt man mit einem Phosphonat der Formel

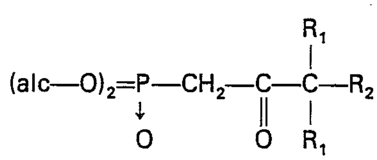

worin $R_1$ und $R_2$ die in Anspruch 1 angegebene Bedeutung besitzen und alc einen Alkylrest mit 1 bis 4 Kohlenstoffatomen bedeutet, in Gegenwart einer starken Base behandelt, um ein Produkt der Formel VII

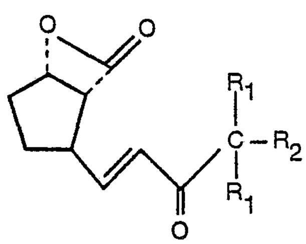

VII

zu erhalten, welches Produkt man mit einem Reduktionsmittel behandelt, um ein Produkt der Formel I zu erhalten.

6. Als Arzneimittel, die Produkte der Formel I gemäß Anspruch 1.

7. Als Arzneimittel, die Produkte der Formel I gemäß einem der Ansprüche 2 oder 3.

8. Pharmazeutische Zusammensetzungen, enthaltend als Wirkstoff zumindest eines der Arzneimittel gemäß einem der Ansprüche 6 oder 7.

**Claims**

1. The products with the general formula (I):

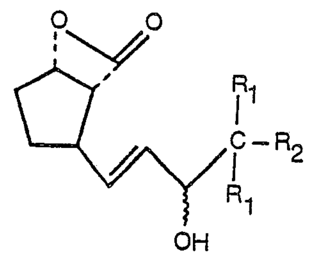

I

in which $R_1$ represents a hydrogen atom or a methyl radical and $R_2$ represents an —X—Ar radical in which X represents an oxygen atom or a methylene radical and Ar represents a phenyl radical, unsubstituted or substituted by one or more radicals chosen from the halogen atoms and the trifluoromethyl radical, the wavy line signifies that the bond may be found in either of the possible configurations.

2. The lactone of (1RS, 2SR, 5RS, 3'RS) (1'E)-2-hydroxy-5-[-3'-hydroxy-4'-phenyloxy-1'-butenyl]cyclopentane-1-carboxylic acid.

3. The lactone of (1RS, 2SR, 5RS, 3'SR) (1'E)-2-hydroxy-5-[-3'-hydroxy-4'-phenyloxy-1'-butenyl]cyclopentane-1-carboxylic acid.

4. Preparation process for the products with the formula (I) as defined in Claim 1, characterized in that a product with the formula (II):

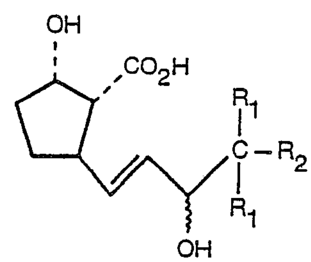

II

in which $R_1$ and $R_2$ have the significance given in Claim 1, is submitted to the action of a reagent for forming functional derivatives of acids, chosen from the group formed by tosyl chloride, alkyl chloroformates, dicyclo-alkylcarbodiimides, dialkylcarbodiimides and thionyl chloride.

5. Preparation process for the products with the formula (I) as defined in Claim 1, characterized in that the product with the formula (III):

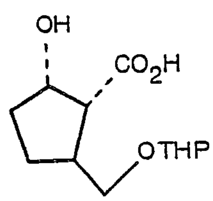

III

in which THP represents a tetrahydropyrannyl radical, is submitted to the action of a reagent for forming functional derivatives of acids, so as to obtain a product with the formula (IV):

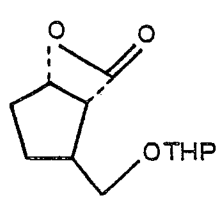

IV

which product is treated with an acid so as to obtain a product with the formula (V):

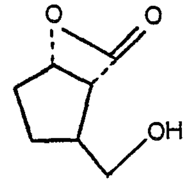

V

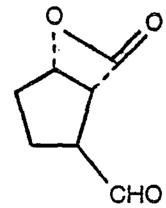

VI

which product is treated with a phosphonate with the formula:

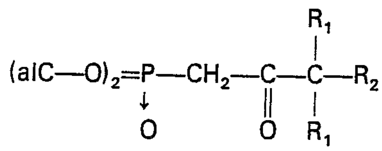

in which $R_1$ and $R_2$ have the significance given in Claim 1 and alk represents an alkyl radical containing 1—4 carbon atoms, in the presence of a strong base, so as to obtain a product with the formula (VII):

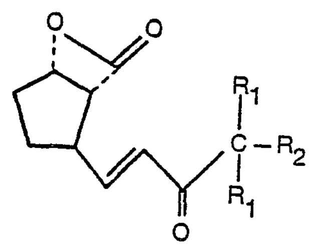

VII

which product is treated with a reducing agent so as to obtain a product with the formula (I).

6. As medicaments, the products corresponding to the formula (I) as defined in Claim 1.

7. As medicaments, the products corresponding to the formula (I) as defined in any one of Claims 2 or 3.

8. The pharmaceutical compositions containing as active principle at least one of the medicaments according to one of Claims 6 or 7.